# EUROPEAN PATENT SPECIFICATION

(11) **EP 4 529 434 B1**
(45) Date of publication and mention of the grant of the patent: **03.06.2026**
(21) Application number: 23749256.6
(22) Date of filing: 29.06.2023
(51) Int. Cl.: A61B 1/00, A61B 1/015, A61M 13/00

(54) **DEVICES AND SYSTEMS FOR REDUCING FLUID VOLUME WHEN USING FLUID TO ACCESS A BODY LUMEN**
VORRICHTUNGEN UND SYSTEME ZUR REDUZIERUNG DES FLÜSSIGKEITSVOLUMENS BEI VERWENDUNG VON FLÜSSIGKEIT ZUM ZUGANG ZU EINEM KÖRPERLUMEN
DISPOSITIFS ET SYSTÈMES POUR RÉDUIRE UN VOLUME DE FLUIDE LORS DE L'UTILISATION D'UN FLUIDE POUR ACCÉDER À UNE LUMIÈRE CORPORELLE

(30) Priority: 30.06.2022 US 202263357615 P
(43) Date of publication of application: 02.04.2025
(73) Proprietor: Boston Scientific Scimed Inc., Maple Grove, Minnesota 55311 (US)
(72) Inventor: CORBEIL, Scott Edward, Litchfield, New Hampshire 03052 (US)
(74) Representative: Vossius & Partner Patentanwälte Rechtsanwälte mbB
(86) International application number: PCT/US2023/069413
(87) International publication number: WO 2024/006932

(56) References cited:
- WO-A1-2020/040651
- US-A1- 2012 116 285
- US-A1- 2019 380 565
- US-A1- 2021 251 654

## Description

### CROSS-REFERENCE TO RELATED APPLICATIONS

This application claims the benefit of US Prov. Pat. App. No. 63/357,615, filed June 30, 2022, titled DEVICES, SYSTEMS, AND METHODS FOR REDUCING FLUID VOLUME WHEN USING FLUID TO ACCESS A BODY LUMEN.

### TECHNICAL FIELD

The present disclosure pertains to medical devices, and methods for manufacturing and use of these medical devices. More particularly, the present disclosure pertains to medical devices for accessing a body lumen along a biliary and/or pancreatic tract.

### BACKGROUND

A wide variety of intracorporeal medical devices have been developed for medical use, for example, for endoscopic procedures. Some of these devices include guidewires, catheters, catheter systems, endoscopic instruments, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

WO 2020/040651 A1 relates to insufflation systems providing a continuous flow of insufflation gas to a body cavity. The continuous flow may be directed over the lens of an endoscope received within a cannula to form a protective envelope around the lens and improve visibility. The continuous flow may be supplied by a pressurized gas source. The continuous flow line may be assembled in parallel to an insufflation line running through a standard insufflator configured to provide noncontinuous gas flow to the body cavity. The lines may converge upstream of or at the cannula or the insufflation flow may be provided to a separate cannula. Continuous gas flow may be provided by recirculating gas from the body cavity through the cannula. Continuous gas flow may be provided by storing gas from the noncontinuous insufflation flow in an accumulator and releasing the gas during off phases of the insufflation flow.

US2019/380565 relates to medical systems for facilitating access to an opening of a body lumen through an endoscope by at least partially dilating the opening with fluid from a fluid source prior to contacting the body lumen. This US patent application discloses sphincterotomes and endoscopic access devices and operator-controlled fluid injection, typically via syringes or pumps.

### SUMMARY

The invention is defined in the appended claims. Aspects, embodiments and examples disclosed herein which do not fall within the scope of the appended claims do not form part of the invention and are merely provided for illustrative purposes.

This disclosure provides design, material, manufacturing method, and use alternatives for medical devices and medical systems.

An example medical device for accessing a body lumen of a subject includes a fluid accumulator having a proximal end configured to receive fluid from a pressurized gas source and a second end, a flexible elongate tube having a proximal end and a distal end configured to be directed toward an opening of a body lumen, a lumen extending from the distal end of the flexible elongate tube toward the proximal end of the flexible elongate tube, wherein the lumen is in selective fluid communication with the second end of the fluid accumulator, and a switch, wherein actuation of the switch is configured to adjust the fluid communication between the fluid accumulator and the lumen.

Alternatively or additionally to any of the examples above, the actuation of the switch is configured to automatically cause fluid to exit a distal end of the lumen at a first rate at a first time and at a second rate at a second time after the first time, the first rate is faster than the second rate.

Alternatively or additionally to any of the examples above, the switch is in communication with a valve that selectively blocks a fluid flow downstream of the fluid accumulator and upstream of the distal end of the lumen.

Alternatively or additionally to any of the examples above, the switch is in mechanical communication with the valve.

Alternatively or additionally to any of the examples above, a first actuation of the switch is configured to cause the fluid accumulator and the lumen to be in fluid communication, and a second actuation of the switch is configured to cause a fluid flow between the fluid accumulator and the lumen to be blocked.

Alternatively or additionally to any of the examples above, the fluid accumulator is a coil of tubing.

Alternatively or additionally to any of the examples above, the medical device may further include a first tubing portion extending between the fluid accumulator and the switch, a second tubing portion extending between the switch and a third tubing portion, and wherein the third tubing portion includes the flexible elongate tube.

Alternatively or additionally to any of the examples above, the medical device may further include a fluid source lumen extending from the fluid accumulator and configured to be in fluid communication with a fluid source, and a flow restrictor positioned along the fluid source lumen.

An example system may include a fluid accumulator and a sphinctertome in fluid communication with the fluid accumulator, and wherein the fluid accumulator is upstream of the sphinctertome and configured to accumulate pressurized fluid for selective release through a distal end of the sphinctertome at an initial first rate that decays over time to a second rate.

Alternatively or additionally to any of the examples above, the system may further include a pressurized fluid source upstream of and in communication with the fluid accumulator to supply pressurized fluid to the fluid accumulator.

Alternatively or additionally to any of the examples above, the second rate is based on a fluid flow rate of the fluid exiting the pressurized fluid source.

Alternatively or additionally to any of the examples above, the system may further include a flow restrictor positioned between the pressurized fluid source and the fluid accumulator to restrict flow from the pressurized fluid source to the fluid accumulator.

Alternatively or additionally to any of the examples above, the system may further include a valve upstream of the sphinctertome and downstream of the fluid accumulator, the valve is configured to adjust a flow of fluid from the fluid accumulator to the sphinctertome.

Alternatively or additionally to any of the examples above, the system may further include a switch in communication with the valve, wherein the switch is configured to be actuated to switch the valve between a closed position in which a fluid flow between the fluid accumulator and the sphinctertome is blocked and an opened position in which fluid flows between the fluid accumulator and the sphinctertome.

An example method of accessing a body lumen of a subject may include extending an elongate tube having a distal end and a lumen through a working channel of an endoscope extended into the subject, directing the distal end of the elongate tube toward an opening of the body lumen, flowing fluid from a fluid source through the lumen and out of the distal end of the elongate tube toward the opening of the body lumen, and wherein flowing fluid from the fluid source through lumen and out of the distal end of the elongate tube includes outputting the fluid from the distal end of the elongate tube initially at a first predetermined rate that automatically decays to a second, non-zero predetermined rate over time.

Alternatively or additionally to any of the examples above, the first predetermined rate is configured to cause the opening of the body lumen to move from a closed state to an opened state.

Alternatively or additionally to any of the examples above, the second, non-zero predetermined rate is configured to cause the opening to remain in an opened state once opened.

Alternatively or additionally to any of the examples above, the second, non-zero predetermined rate is maintained for a time period that is longer than a time period at which the fluid is outputted at the first predetermined rate.

Alternatively or additionally to any of the examples above, the method may further include opening a valve to cause fluid to flow from the fluid source through the lumen and out of the distal end of the elongate tube.

Alternatively or additionally to any of the examples above, the method may further include closing the valve to cause fluid from the fluid source to accumulate in a fluid accumulator in selective fluid communication with the lumen.

The above summary of some embodiments is not intended to describe each disclosed embodiment or every implementation of the present disclosure.

### BRIEF DESCRIPTION OF THE DRAWINGS

The disclosure may be more completely understood in consideration of the following detailed description in connection with the accompanying drawings, in which:
FIG. 1 is a schematic view of an illustrative system for accessing an opening of a body lumen of a subject;
FIG. 2 is a schematic view of the system depicted in FIG. 1, taken from circle-2;
FIG. 3 is a schematic view of an illustrative system for accessing a body lumen;
FIG. 4 is a schematic view of an illustrative system for accessing a body lumen, where body tissue and an endoscopic device are shown in cross-section;
FIG. 5 is a schematic view of an illustrative system for accessing a body lumen;
FIG. 6 is a schematic view of an illustrative system for accessing a body lumen;
FIG. 7 is a schematic diagram of an illustrative method of using a system for accessing a body lumen; and
FIGS. 8-10 are schematic views of an illustrative system for accessing a body lumen of a subject being used to access the body lumen.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure.

### DETAILED DESCRIPTION

For the following defined terms, these definitions shall be applied, unless a different definition is given in the claims or elsewhere in this specification.

Although configurations of the present disclosure may be described with specific reference to medical devices and systems (e.g., endoscopic devices, accessory tools, and/or guidewires inserted through a duodenoscope or endoscope, near or through a papilla, or the like) for selective access to, aligning with, and/or cannulation of the common bile duct (CBD) or pancreatic duct (PD) during an Endoscopic Retrograde Cholangiopancreatography (ERCP) procedure, it should be appreciated that such medical devices and systems may be used in a variety of medical procedures which require navigating one or more accessory tools through ductal, luminal, vascular, or body lumen anatomies, including, for example, interventional radiology procedures, balloon angioplasty/angiography procedures, thrombolysis procedures, urological or gynecological procedures, and the like. The disclosed medical devices and systems may be inserted via different access points and approaches, e.g., percutaneously, endoscopically, laparoscopically, or some combination thereof.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5). Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The detailed description is intended to illustrate but not limit the disclosure. Those skilled in the art will recognize that the various elements described may be arranged in various combinations and configurations without departing from the scope of the disclosure. The detailed description illustrates example embodiments of the disclosure.

As discussed herein, it may be desirable for a distal tip of a medical device to be flexible to navigate effectively through a body lumen. For example, flexible distal tips of medical devices may be capable of facilitating navigation through narrow passages such as the papilla of Vater and/or other passages. In some instances, a flexible distal tip of a medical device may facilitate steering the medical device into a target body lumen that is closely situated to structures such as lesions, stones or other build-up and/or has such structures situated therein.

In some instances, the devices and methods that are disclosed herein may be useful for diagnostic or therapeutic procedures in the biliary and/or pancreatic tracts, among being useful for other purposes. Access to the pancreaticobiliary system, as facilitated by the devices disclosed herein, may be required to diagnose and/or treat a variety of conditions, including but not limited to tumors, gallstones, infection, sclerosis, and pseudo cysts. The devices disclosed herein may also be useful for navigation in other parts of the body such as the cardiovascular system and so forth.

Endoscopic retrograde cholangiopancreatography (ERCP) may be used to diagnose and treat conditions of the common bile duct, including, for example, gallstones, inflammatory strictures, leaks (e.g., from trauma, surgery, etc.), cancer, and/or other conditions. In an ERCP procedure, a physician may view, through an endoscope, the inside of the stomach and/or the duodenum. During the procedure, dyes may be injected into the ducts in the biliary tree and/or pancreas so that the area can be seen using X-rays. These procedures may necessitate gaining and keeping access to the papilla of Vater, the common bile duct, and/or the pancreatic duct, which may be technically challenging, may require extensive training and practice to gain proficiency, and may require one or more expensive tools in order to perform.

During an ERCP procedure, a number of steps may be performed while the subject is sedated and/or anaesthetized. For example, an endoscope may be inserted through the mouth, down the esophagus, into the stomach, through the pylorus into the duodenum, and to a position at or near the papilla of Vater (also referred to as the ampulla of Vater), which is the opening of the common bile duct and the pancreatic duct. Due to the shape of the papilla, and the angle at which the common bile duct and pancreatic duct meet the wall of the duodenum, the distal end of the endoscope is generally placed just past the papilla. Due to the positioning of the endoscopes beyond the papilla, the endoscopes typically used in these procedures are side-viewing endoscopes. The side-viewing feature provides imaging along the lateral aspect of the distal end rather than from an axial aspect at a terminal end of the endoscope. Such orientation may allow a clinician to obtain an image of the medial wall of the duodenum, where the papilla of Vater is located, even though the distal tip of the endoscope is beyond the opening.

In some cases, a fluid (e.g., a compressible fluid, such as a compressible gas, air, nitrogen, carbon dioxide, etc., and/or other suitable fluid) may be delivered via catheter, endoscope (e.g., as inserted through a subject as discussed above or otherwise inserted), an endoscopic accessory device, or other suitable elongated medical device to an opening (e.g., the papilla of Vater) of a body lumen of a subject. Applying the fluid to the opening may facilitate identifying and/or accessing the body lumen by at least partially dilating the opening with the fluid prior to and/or during delivery of a catheter, endoscope, guidewire, etc. to a target anatomical structure or tissue (e.g., a duct or other suitable structure or tissue) at or distal of the opening of the body lumen. Although a stream of fluid may be used to identify and/or dilate the opening to the lumen, it may be desirable to limit or reduce a total amount of fluid delivered to the body lumens, spaces, or cavities of the subject during a procedure.

Fluid can be delivered via catheter, endoscope, etc. to an opening of a body lumen in any suitable manner. Some example techniques and systems for delivering fluid to an opening of a body lumen are described in U.S. Patent Application Publication No. 2019/0380565, which was filed on June 13, 2019, and titled DEVICES, SYSTEMS AND METHOD FOR ACCESSING A BODY LUMEN.

Systems for delivering compressible fluid to an opening of a body lumen may result in delivery of a volume of gas to an enclosed cavity of a subject. In some cases, the subject's body may require extended periods of time to fully absorb the gas. The volume of gas may leave the subject feeling pain, discomfort, and/or an abnormal sensation until the gas has been fully absorbed or otherwise passed. The volume of fluid provided to the subject may result in tissue barotrauma, disruption, rupture, and/or have other undesirable effects on body tissue of the subject.

As such, it may be desirable to provide a system that is able to facilitate opening a body lumen and maintaining the body lumen in an opened state, while limiting an amount of a fluid provided to the subject. Such a system may be configured to deliver a high initial flow rate of fluid to the subject to facilitate identifying and/or dilating an opening of a body lumen of the subject and then, deliver a lower flow rate of fluid to the subject to facilitate maintaining the body lumen in an opened state. Such a system may reduce or mitigate an amount of fluid needed to open the body lumen and maintain the body lumen in the opened state. Such reduction in total fluid volume provided to a body lumen may reduce the likelihood of causing tissue desiccation, disruption, and/or other damage that may be a result of using high volume and/or high velocity gas when dilating tissue.

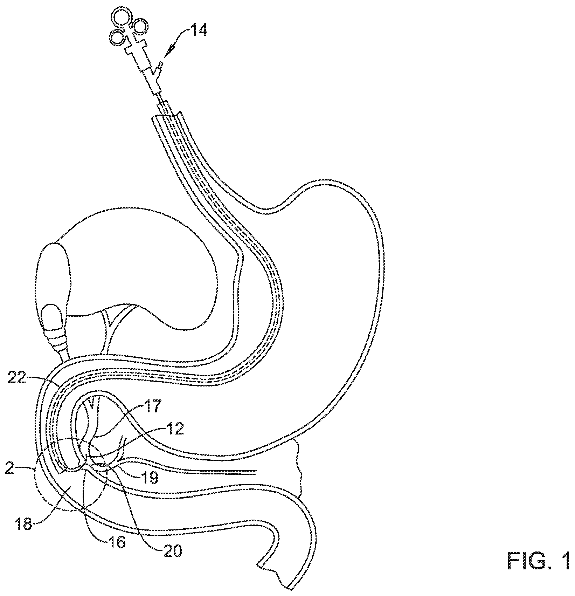

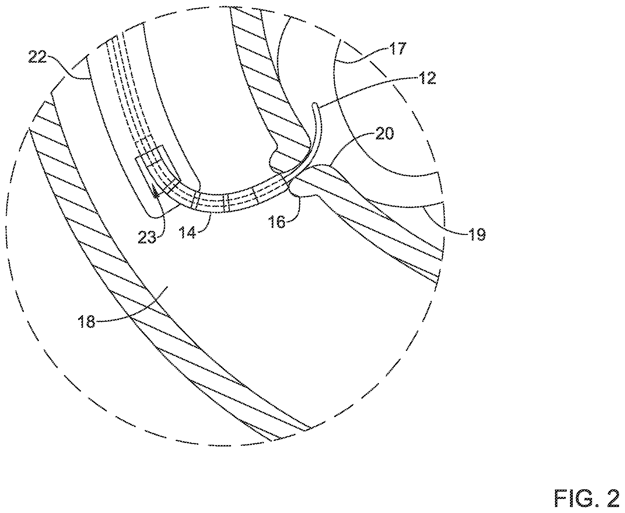

Turning to the Figures, FIGS. 1 and 2 depict a selective cannulation during an ERCP procedure, which includes positioning a distal portion of a medical device 22 (e.g., a duodenoscope, an endoscope, etc.) within the descending duodenum 18 and passing a guidewire 12 and/or an endoscopic accessory device 14 through the medical device 22 towards, against, and/or through a body lumen, such as the major papilla 16 (e.g., ampullary entry) near the descending duodenum 18 to access the Sphincter of Oddi Complex 20. The guidewire 12 and the endoscopic accessory device 14 may be advanced through a working channel 23 of the medical device 22 towards the major papilla 16. Additionally, the guidewire 12 and/or the endoscopic accessory device 14 may be advanced against and/or through the major papilla 16 to a desired one of the common bile duct 17, the pancreatic duct 19, and/or other suitable body lumens.

Accessing the papilla 16 may be difficult because the opening is small compared to a size (e.g., a diameter) of many medical devices, the opening may be completely collapsed/closed, and/or the opening may extend into the descending duodenum 18 at an angle that may be difficult to visualize and/or access. Thus, a medical professional may be required to manipulate the endoscopic accessory device 14 and guidewire 12 by manually rotating the medical device 22 and/or using an elevator within the distal end of the medical device 22 in an attempt to better align or orient the endoscopic accessory device 14 and/or the guidewire 12 with respect to the medical device 22 and the opening of the papilla 16. Difficult cannulation procedures in which the medical professional fails to access the Sphincter Papillae within a certain time limit, or after a certain number of unsuccessful attempts may lead to significant post-procedure complications, such as post-ECRP pancreatitis (PEP).

A medical professional accessing a body lumen (e.g., a duct, such as a papilla, or the like) by manipulating a medical device against or into the opening of the body lumen, as discussed above, may subject the walls of the opening of the body lumen to compressive forces. Compression of a body lumen opening or the body lumen itself can cause buckling in what may be described as the "accordion effect". Should a medical device be inserted into a body lumen without the accordion effect, opening and/or maintaining patency of the body lumen through repeated inserting of the medical device and/or a guidewire or other instruments may cause irritation to the body tissue from friction.

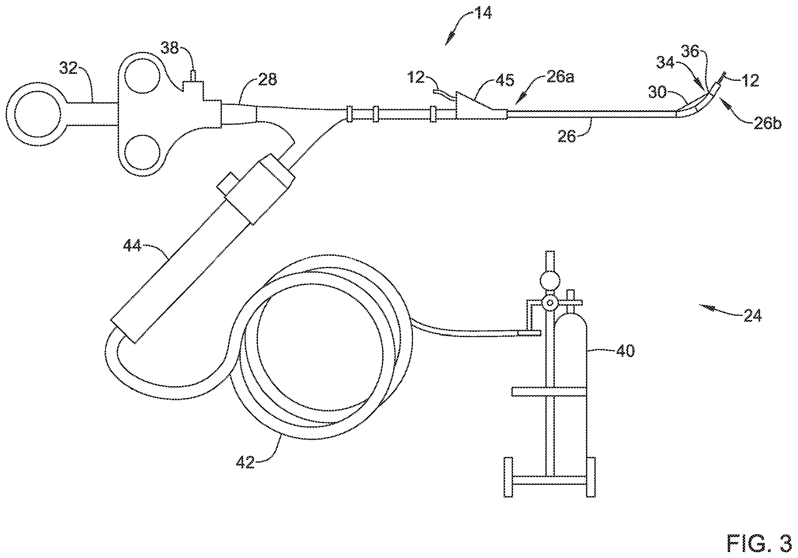

FIG. 3 depicts an illustrative system 24 for accessing a body lumen without requiring contact from a medical device to open an opening to the body lumen and maintain the opening in an open state during a procedure and/or otherwise when positioning medical devices in the body lumen. In some cases, the system 24 may include, among other components, the medical device 22 with a flexible elongate tube 26.

The tube 26 may have a proximal end 26a and a distal end 26b. The distal end 26b of the tube 26 may be configured to be directed toward and/or through an opening of a body lumen. The proximal end 26a of the tube 26 may be connected to, coupled with, or otherwise in communication with a handle 28.

A wire filament 30 may extend along the elongate tube 26 and may be energized for the purpose of cutting tissue within a subject. The wire filament 30 may have a proximal end (not shown) connected to an actuating portion 32 of the handle 28, and a proximal portion extending at least partially within the tube 26. The wire filament 30 also may have a distal portion 34 extending external to the elongate tube 26, and a distal end 36 of the filament 30 may be connected to the distal end 26b of the elongate tube 26.

The wire filament 30 may be received within a lumen of the tube 26 at least along a proximal portion of the filament 30, and may be configured to slide within the lumen. In some cases, the actuating portion 32 of the handle 28 may be manipulated to slide the wire filament 30 along the tube 26 (e.g., within the lumen of the tube 26).

Sliding the wire filament 30 may result in the distal end 26b of the elongate tube 26 moving responsively to the sliding of the wire filament 30. As such, the actuating portion 32 of the handle 28 may be manipulated to control or adjust a position of the distal end 26b of the elongate tube 26. In one example, the actuating portion 32 of the handle 28 may be manipulated to direct the distal end 26b of the tube 26 toward the opening of a body lumen.

In some cases, the wire filament 30 may be utilized to electrically cut, remove, and/or cauterize tissue of the subject. As such, the handle 28 or other suitable portion of the medical device 22 may include an electrical connection 38 for an energy source (e.g., a radiofrequency energy source, or the like) to energize the wire filament 30 and facilitate cutting tissue. When the actuating portion 32 of the handle 28 is manipulated to apply tension to the wire filament 30 and adjust a position of the distal end 26b of the tube 26, the wire filament 30 may be spaced from the tube 26 and electrified to facilitate cutting tissue at or adjacent to the opening of the body lumen.

The medical device 22 may include one or more lumens extending from at least the proximal end 26a of the tube 26 to the distal end 26b of the tube 26. Although not required, at least a portion of the one or more lumens may move along with the distal end 26b (e.g., toward an opening of a body lumen) of the tube 26.

In some cases, a first lumen of the one or more lumens of the elongate tube 26 may have a fluid connection at a proximal end for a pressurized fluid source 40 (e.g., a CO₂ tank or a container of other suitable fluid). The first lumen may be configured to apply a fluid (e.g., a gas) from the fluid source 40 to an opening at a body lumen of the subject to open or at least partially open the opening for access into the body lumen by the guidewire 12 and/or the elongate tube 26 without or substantially without the elongate tube 26 contacting the body lumen. Once the body lumen is opened or partially opened, a user may energize the wire filament 30 to cut or remove body tissue to further enlarge the opening.

A second lumen of the one or more lumens may be configured to accept a guidewire 12. The second lumen may extend from the distal end 26b of the tube 26 at least partially along the tube 26 toward the proximal end 26a of the tube 26. In some cases, the guidewire 12 may extend proximally out of an aperture port 45 and/or distally out of a distal opening at the distal end 26b of the tube 26. Once positioned in a target body lumen, the tube 26 may be withdrawn from the guidewire 12 and the guidewire 12 may be used to guide the medical device 22 or other instruments to a target area in the body lumen of the subject.

The aperture port 45 may be located at the distal end of the handle 28, the proximal end 26b of the tube 26, and/or at one or more other suitable locations. In some cases, the aperture port 45 and the tube 26 may be configured so that the guidewire 12 may be stripped through the side of the tube 26 for a rapid removal or exchange of devices, but this is not required.

A third lumen of the one or more lumens may be configured to be in fluid communication with a source of a contrast agent and to deliver the contrast agent to the body lumen and/or associated tissue of the subject. The contrast agent may be supplied to the third lumen via a syringe attached to a luer connector on the handle 28, for example, and/or through one or more other suitable connections. The contrast agent may be delivered into the body lumen and may be used to temporarily improve imaging of the inside of the body lumen by, for example, x-ray, fluoroscopy, computed tomography (CT), or magnetic resonance (MR) imaging, ultrasound, and the like.

Although not required, a filter (e.g., a 0.1 micro filter and/or other suitable filter) in a tubing 42 may be in fluid communication with the first lumen distal to or downstream of the fluid source 40 and proximal to or upstream of the first lumen. In some cases, a flow regulator 44 may be in fluid communication with the first lumen and may be utilized to regulate a flow of fluid from the fluid source 40, through the filter (when included) in the tubing 42, and to or within the first lumen of the tube 26.

The flow regulator 44 may include a valve configured to allow a user to vary the flow rate and/or pressure of the fluid through the device 22. In some configurations, the flow regulator 44 may be or may include an on/off valve. In one example, the flow regulator 44 may be or may include a trumpet valve.

A relief hole and/or valve may be located along the fluid path from the fluid source 40 and upstream of the elongate tube 26 to release pressure from the system 24 if the pressure of the fluid becomes too high for the procedure or for the patient's safety due to, for example, a blockage in a lumen or perhaps a failure of the regulator 44. The relief hole may include a one-way valve that opens at a certain threshold of pressure and/or may have one or more other suitable configurations.

In some cases, a pressure of the fluid at the fluid source 40 may be higher than a desired pressure of the fluid at the accessory device 14. As such, fluid from the fluid source 40 may be fed through the regulator 44 to reduce the fluid pressure before the pressure relief hole or valve. The regulator 44 and/or the relief valve may protect the subject and accessory device 14 from the higher pressure of the fluid source 40 by down-regulating the pressure at the regulator 44 or providing a relief threshold at the relief hole or valve to relieve fluid pressure if pressure at the hole or valve exceeds the relief threshold.

In some cases, the pressure relief hole or valve may be located downstream of the flow regulator 44. In such cases, if the regulator 44 fails or is set incorrectly, the pressure relief valve may open to prevent an undesirably high pressure and/or high volume of fluid from entering the accessory device 14. A range of suitable fluid pressure for the system or device delivered from the downstream side of the regulator 44 and/or the relief valve may be, for example, about 10 psi to about 50 psi, and/or other suitable range of pressure values.

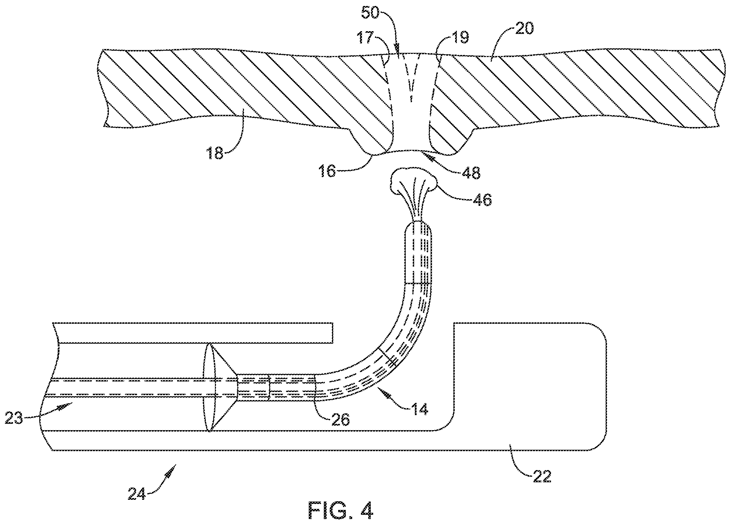

FIG. 4 depicts an enlarged distal end of the system 24 providing fluid 46 to an opening 48 (e.g., an opening defined by the major papilla 16) of a body lumen 50 (e.g., a body lumen defined by the sphincter of Oddi 20). Although not depicted, the fluid 46 may come from a fluid source (e.g., the fluid source 40 and/or other suitable fluid source) and exit the distal end of the tube 26 at a constant or substantially constant velocity and/or pressure to adjust the opening 48 to an opened state and maintain the opening 48 in an opened state.

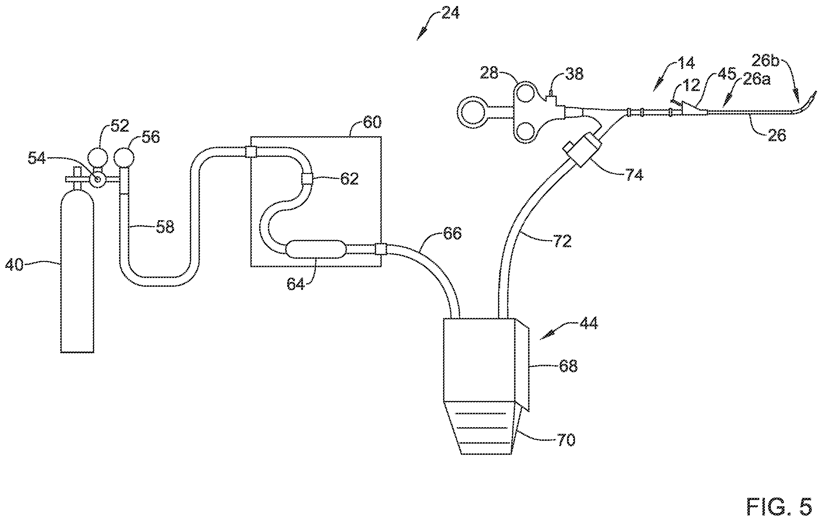

To reduce an amount of fluid provided to a subject and an amount of time a subject receives high pressure and/or flow rate fluid during a procedure, the system 24 may be configured to provide fluid to the subject at a non-constant flow. For example, the system 24 may be configured to provide fluid to the subject at an initial flow rate or exit velocity and then ramp down the flow rate or exit velocity at which the fluid is provided to the subject until the flow reaches a desired flow rate or exit velocity of the fluid. In some cases, the desired flow rate or exit velocity of the fluid is a flow rate or exit velocity that is needed to maintain the opening of the body lumen in an opened state after initially using the fluid to adjust the opening from a closed state to the opened state. The ramping down of flow rate or exit velocity of fluid provided to the subject from a first flow rate or exit velocity to a lower, second flow rate or exit velocity may be done in a linear manner, a step-wise manner, and/or one or more other suitable manners. Figures 5-10 depict systems, components, and methods for providing fluid to a subject during a procedure at reduced flow rate or velocity over time until a predetermined flow rate or velocity is reached.

FIG. 5 depicts a configuration of the system 24 set up to provide fluid to a subject at a first flow rate or exit velocity (e.g., an initial flow rate or exit velocity), where the flow rate or exit velocity of the fluid provided to the subject may be ramped down over time (e.g., decay) to a second flow rate or exit velocity. In some cases, the second flow rate or exit velocity may be based, at least partially, on a fluid flow rate or exit velocity of the fluid exiting the pressurized fluid source. The first flow rate or exit velocity may be greater than the second flow rate or exit velocity.

Starting from a proximal end of the system 24, the system 24 depicted in FIG. 5 may include, among other components, one or more fluid sources 40 (e.g., a cylinder or container containing pressurized fluid), one or more cylinder pressure gauges 52 configured to provide an indication of pressure in the one or more fluid sources 40, one or more pressure controllers 54 (e.g., a pressure adjustment knob and/or other suitable pressure controller) configured to control a pressure or flow rate (e.g., volumetric flow rate) of fluid out of the one or more fluid sources 40, one or more outlet pressure gauges 56 configured to provide an indication of pressure or flow rate of fluid exiting the one or more fluid sources 40 downstream of the one or more pressure controllers 54, fluid source outlet tubing 58, a pressurizing system configured to receive fluid from the fluid source outlet tubing 58 and including a flow restrictor 62 and a fluid accumulator 64, accumulator outlet tubing 66 in communication with an outlet of the fluid accumulator 64 and configured to receive fluid from the fluid accumulator 64, the flow regulator in communication with the accumulator outlet tubing 66 and configured to receive fluid from accumulator outlet tubing 66, and flow regulator outlet tubing 72 in communication with the flow regulator 44 and a connector 74 (e.g. a luer connector and/or other suitable type of connector) of the endoscopic accessory device 14 (e.g., a sphinctertome and/or other suitable accessory device) to provide fluid passing through the flow regulator 44 to the accessory device 14 to cause an opening of a body lumen of a subject to open and remain open over time. A lumen of the elongate tube 26 of the accessory device 14 may extend from the distal end of the elongate tube 26 toward the proximal end of the elongate tube 26 and through the connector 74 to the flow regulator outlet tubing 72 that is in selective communication with the fluid accumulator 64 via a flow valve 68 (e.g., within a housing, as shown in the Figures).

Although multiple tubes 58, 66, 72 are labeled in FIG. 5 and discussed above, a single tube or more than one tube may be utilized, as desired. In some cases, the tubing of the system 24 may be combined to form a single lumen or multiple lumens through which fluid from the fluid source 40 passes to and through the accessory device 14 to the subject.

The pressure controller 54 may be adjusted to control a pressure or flow rate of fluid exiting the fluid source 40. In some cases, when included, the fluid source pressure gauge 52 may display or otherwise provide an indication of the pressure upstream (e.g., in the fluid source 40) of the pressure controller 54. When included, the fluid source outlet pressure gauge 56 may display or otherwise provide an indication of the pressure or flow rate of fluid downstream of (e.g., output from) the pressure controller 54, as set by the pressure controller 54 and at which fluid is provided to the pressurizing system 60.

The pressurizing system 60 may be configured to facilitate providing fluid to the subject at a pressure or flow rate at or less than a pressure or flow rate set by the pressure controller 54 and may include the flow restrictor 62 and the fluid accumulator 64. In some cases, the pressurizing system 60 may include a housing that houses, the flow restrictor 62, the fluid accumulator 64, and/or other suitable components of the pressurizing system 60, but this is not required. The flow restrictor 62 may be configured to narrow a flow path of the fluid to limit a flow rate of the fluid traveling through the flow path given an inlet pressure from the fluid source 40. The flow restrictor 62 may be fixed or optionally may be variable. Other suitable flow restrictors 62 are contemplated. In some cases, the flow restrictor 62 may be omitted.

The fluid accumulator 64 may have a proximal end configured to receive fluid from the pressurized fluid source 40 and a second end (e.g., a distal end). As depicted in FIG. 5, the fluid accumulator 64 may be a cylinder configured to accumulate fluid when the flow regulator 44 is in a closed state. Other suitable types of fluid accumulators are contemplated including, but not limited to coils of tubing, a length of tubing, one or more tanks, a chamber in-line with the tubing or flow path of the fluid of the system 24 and/or other suitable volumes that are configured to add compressing fluid volume between the flow restrictor 62 and the flow regulator 44 (e.g., that are configured to be filled with pressurized fluid) when the flow regulator 44 is in a closed state and output the pressurized fluid when the flow regulator 44 is switched to an opened state.

The fluid accumulator 64 may be any suitable type of fluid accumulator. In some cases, the fluid accumulator 64 may be a fixed volume. In such cases, when pressurized fluid at a constant rate is supplied to the accumulator 64 and flow regulator 44 is opened, a flow rate of fluid provided to the subject may linearly decay over time from an initial flow rate to a steady state flow rate.

The flow regulator 44 may include a flow valve 68 and a switch actuator 70 (e.g., a switch). The flow valve 68, as depicted in the Figures, may be within a housing and configured to regulate a flow from the accumulator outlet tubing 66 through the flow regulator outlet tubing 72 (e.g., regulate a flow through a lumen or lumens of the tubing 66, 72). The tubing 66, 72 and/or the lumen(s) thereof may be coupled with and/or communicate with the flow valve 68 in any suitable manner such that actuation of the flow valve 68 adjusts a flow through the lumen(s) of the tubing 66, 72.

The flow valve 68 may be any suitable type of valve in communication with a flow path of the pressurized fluid from the fluid source 40 to the accessory device and adjustable between an opened state and a closed state. When the flow valve 68 is in the closed state, the fluid accumulator 64 may accumulate pressurized fluid for release under pressure when the flow valve 68 is adjusted to an open state or position.

In some configurations, the flow valve may be an on/off valve. Additionally or alternatively, the flow valve 68 may be an adjustable valve having an off configuration that blocks flow through the lumen(s) of the tubing 66, 72, and an adjustable "on" configuration allowing for different, adjustable levels of flow through the lumen(s) of the tubing 66, 72. In one example configuration, the flow valve 68 may be or may include a trumpet valve, but this is not required.

The switch or switch actuator 70 may be in mechanical, electromechanical, and/or electrical communication with the flow valve 68, such that the switch actuator 70 may be adjusted to adjust the flow valve 68 between an opened state and a closed state. In one example, a first actuation of the switch actuator 70 may cause the fluid accumulator 64 and the lumen of the accessory device 14 to be in fluid communication and a second or subsequent actuation of the switch actuator 70 may cause a fluid flow between the fluid accumulator 64 and the lumen of the accessory device 14 to be blocked.

The switch actuator 70 may be any suitable type of actuator configured to be adjusted to switch a state of the flow valve 68. As depicted in FIG. 5, the switch actuator 70 may be a foot pedal. Alternatively or additionally, the switch actuator 70 may be a button, a dial, a knob, a touch screen controller, an on/off switch, and/or other suitable type of switch actuator 70.

In operation, the system 24 may be utilized to enable fluid to accumulate or pressurize in the fluid accumulator 64, such that when the switch actuator 70 is adjusted or actuated to cause the flow valve 68 to adjust from a closed state to an opened state, pressurized fluid may be delivered at a relatively high flow rate from the fluid source 40 and the fluid accumulator 64 to the subject through a lumen of the accessory device 14 at a relatively high exit velocity. The first flow rate may be determined by the source pressure set at the pressure controller 54, which may be the maximum pressure achieved within the fluid accumulator 64 when the flow valve is in a closed state. As such, when the fluid accumulator 64 is positioned downstream of the flow restrictor 62, has a defined volume, and is pressurized by the fluid from the fluid source 40, a maximum value of the flow rate of the fluid exiting the fluid accumulator 64 may be determined by the first pressure in the accumulator 64 and the inherent flow resistance of the flow path down stream of fluid accumulator 64. With the flow valve 68 in the opened state, fluid flow from the fluid accumulator 64 flows out of the fluid accumulator 64 faster than fluid can enter the fluid accumulator 64 due to the flow restrictor 62 between the fluid source 40 and the fluid accumulator 64, and thus, the initial relatively high flowrate and fluid exit velocity of the fluid from the distal end 26b of the elongate tube 26 ramps down over time until it reaches a steady state flow rate or exit velocity (e.g. a second flow rate or velocity) dictated by 1) a pressure set at the pressure controller 54 and 2) a configuration of the flow restrictor 62, among other factors. The second flow rate or velocity may be considered a steady state flow rate or velocity, which occurs when fluid is entering the fluid accumulator 64 at the same rate as it is exiting the fluid accumulator 64. Then, once the flow valve 68 closes or starts to close, the system 24 may reset itself and pressurized fluid will again fill the fluid accumulator 64, such that the process of supplying high pressure or flow rate fluid to subject may be repeated, as needed.

A configuration of the system 24 may be configured and/or adjustable to obtain desired flow rates, velocities, or pressures of fluid provided to the subject over time. For example, a configuration (e.g., a size, shape, etc.) of the fluid accumulator 64, a fluid pressure set at the pressure controller 54, and a configuration (e.g., a size, shape, etc.) of the flow restrictor 62 may be configured or adjustable such that a peak flow rate (e.g., an initial or first flow rate), a flow rate decay rate (e.g., a slope from the peak flow rate to the steady state flow rate), a ramp duration (e.g., a transition time from peak pressure or flow rate to a steady state pressure or flow rate), and/or a steady state pressure or flow rate (e.g., a second flow rate) may be set to desired levels (e.g., levels sufficient to open an opening at a body lumen, maintain an opening in an opened state, provide not more than a desired amount of fluid to the subject, etc.). In some cases, among other factors, a value of the fluid pressure set at the pressure controller 54 may determine the peak or first flow rate or exit velocity, the fluid pressure and/or flow rate set at the pressure controller 54 and a configuration of the flow restrictor 62 may determine the steady state or second flow rate or exit velocity, and a size of the fluid accumulator 64 and characteristics of the fluid pathway downstream of the accumulator 64 may determine a pressure or flow rate decay rate and/or a duration between the peak or first flowrate or exit velocity and the steady state or second flow rate or exit velocity.

The system 24 depicted in FIG. 5 may allow for delivery of gas at a relatively high initial flow rate or exit fluid velocity from the accessory device 14 when activating or opening the flow valve 68 to facilitate dilating or otherwise opening a body lumen opening and then, automatically reduce the flow rate or exit velocity of the fluid delivered to the subject to a level sufficient to maintain the opening of the body lumen to allow for placing of one or more medical devices within the body lumen. The ramp down in flow rate may result in significantly lower total gas volume being delivered to a subject during a procedure relative to existing systems that deliver fluid to a subject for the purpose of opening body lumens. The reduction in total gas volume delivered to the subject may reduce risks associated with distending and pressurizing the closed or normally closed body lumens of the subject in which a procedure is taking place. Further, ramping down the fluid flow rate or pressure may reduce an overall stress applied to impacted tissues and structures over the time the pressurized fluid is delivered to the subject and may also reduce a risk of adventitious effects such as tissue desiccation and/or other effects, due to tissue being exposed to a volume of dry fluid expanding from a compressed source.

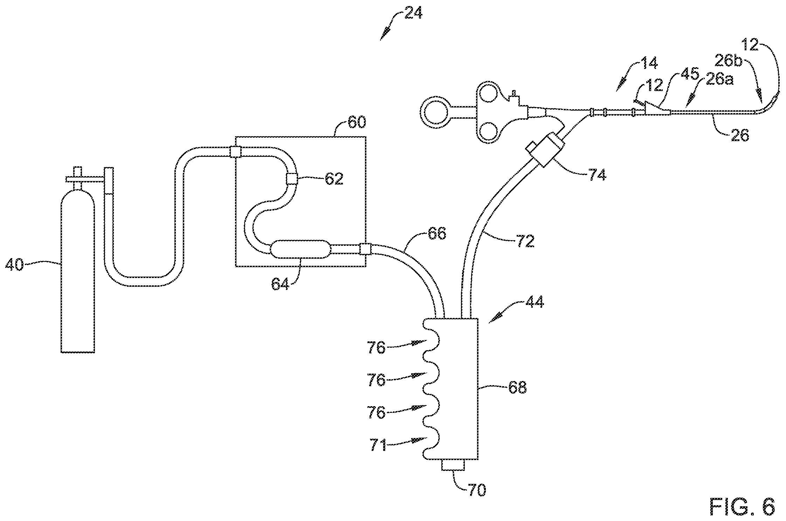

FIG. 6 depicts a further configuration of the system 24 that operates in a manner similar to the configuration of the system 24 discussed above with respect to FIG. 5, where the configuration of the system 24 in FIG. 6 has a pressurizing system 60 that includes the fluid source 40, the flow restrictor 62, and the fluid accumulator 64. In some cases, the pressurizing system 60 may include a housing that houses the fluid source 40, the flow restrictor 62, the fluid accumulator 64, and/or one or more other suitable components of the pressurizing system 60.

When the pressurizing system 60 is configured as depicted in FIG. 6, the pressurizing system may have a releasable connection with the accumulator outlet tubing 66. In such a configuration, the pressurizing system 60 may be detached from the accumulator outlet tubing 66 and may be reused for subsequent procedures on other subjects, while allowing for the tubing 66, 72 and the flow regulator 44 to be disposed of.

Further, as depicted in FIG. 6, the flow regulator 44 may be configured to be handheld. Although FIG. 5 depicts a flow regulator 44 configured as a foot pedal and FIG. 6 depicts a handheld flow regulator 44, the configurations of the system 24 depicted in FIGS. 5 and 6 may use a foot pedal flow regulator 44, a handheld flow regulator 44, and/or other suitably configured flow regulators 44.

In one configuration of a handheld flow regulator 44, the flow valve 68 may include a plurality of locations 76 configured to receive a user's fingers (e.g., a grip location) when the flow valve 68 is grasped. Further, the switch actuator 70 may be configured as a button (as shown in FIG. 6), a trigger, and/or other suitable actuator when the flow valve 68 configured to be grasped by a user's hand. In operation, a user may grasp the flow valve 68 such that their fingers wrap around the flow valve 68 and may actuate the switch actuator 70 by pressing the switch actuator 70 with a thumb of the hand grasping the flow valve 68 or in one or more other suitable manners to facilitate providing a desired flow of pressurized fluid to the subject, but other configurations are contemplated.

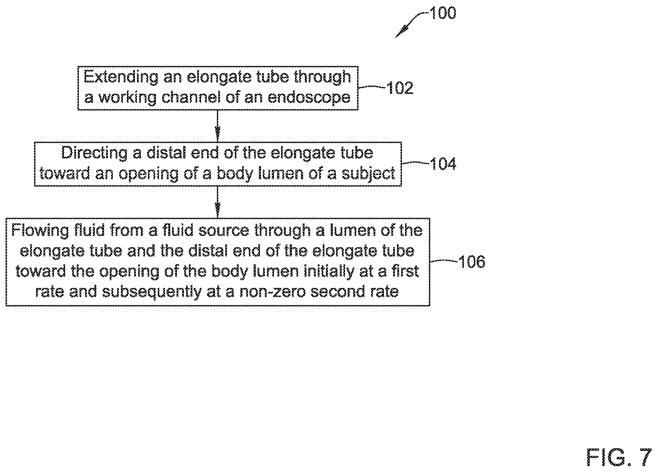

FIG. 7 is a schematic flow diagram of an illustrative technique 100 for accessing a body lumen of a subject. In some cases, the system 24 or a system similar to the system 24 discussed above may be utilized for accessing the body lumen of the subject. Although the technique 100 may be utilized for accessing openings of other body lumens, the technique 100, in one example, may be utilized for accessing the major Papilla of a subject for entrance into the common bile duct and/or the pancreatic duct.

The illustrative technique 100 may include extending 102 an elongate tube of an accessory device (e.g., the elongate tube 26 of the accessory device 14, such as a sphincterotome and/or other suitable elongate tube) through a working channel of an endoscope inserted into a lumen of a subject (e.g., a lumen of the duodenum of the subject and/or other suitable body lumen). The endoscope may be inserted into the lumen using any suitable known technique. The elongate tube may be extended through the working channel of the endoscope prior to or after the endoscope has been positioned in the lumen of the subject, as desired.

A distal end of the elongate tube extending through the working channel of the endoscope may be directed 104 toward an opening of a body lumen of a subject. In some cases, advancing the distal end of the elongate tube through the endoscope after the endoscope has been positioned proximate the opening of the body lumen, may automatically result in the distal end of the elongate tube being directed toward the opening of the body lumen. For example, a visualization system associated with the endoscope may be utilized to position an opening at the distal end of the endoscope proximate the opening of the body lumen. Further, the endoscope may have a ramp feature or other elevator feature to guide the elongate tube out of the opening in the endoscope such that a distal opening of the elongate tube that is in communication with a fluid lumen is pointed at or near the opening of the body lumen. Alternatively or additionally, the elongate tube may include or may be coupled to a wire that can be tensioned at a proximal end to cause the distal end of the elongate tube to bend in a predetermined direction to control a location of the distal opening of the elongate tube as the distal end exits the endoscope.

Further, the technique 100 may include flowing 106 fluid from a fluid source (e.g., the fluid source 40 and/or other suitable fluid source) through the lumen of the elongate tube and out of the distal opening of the elongate tube toward the opening of the body lumen. The flow of fluid from the fluid source through the lumen of the elongate tube may be initiated by adjusting or actuating a switch controller (e.g., the switch actuator 70 or other suitable switch controller) in communication with a valve (e.g., the valve 68 or other suitable valve) to open the valve and allow pressurized fluid to flow from an accumulator and the fluid source to a lumen of the elongate tube and to the subject. The accumulator may be in selective fluid communication with the lumen of the elongate tube via the flow valve and actuation or adjustment of the valve (e.g., in response to actuating the switch controller) may automatically cause fluid to flow from the accumulator and out of the lumen of the elongate tube.

Once the valve is in an opened state, the pressurized fluid from the fluid source and the accumulator may flow to or toward the opening of the body lumen at an initial first predetermined flow rate and subsequently at a non-zero second predetermined flow rate. The flow rate of the fluid to or toward the opening of the body lumen may automatically decay from the first predetermined flow rate to the second predetermined flow rate. Although not required, one or more of the configurations of the system 24 described herein may be utilized to provide a fluid to an opening of a body lumen at an initial predetermined fluid flow rate, which automatically decays to a second predetermined flow rate with a linear decay.

Further, after exhausting all or at least some of the fluid from the accumulator, adjusting the switch controller to cause the valve to close may prevent fluid from the fluid source and the accumulator from reaching or otherwise flowing to the accessory device. As such, the closing of the valve may cause fluid from the fluid source to accumulate in the fluid accumulator, which results in the system "re-charging" so as to be able to provide fluid at the first predetermined flow rate a second or subsequent time.

The first predetermined flow rate or pressure of the fluid provided to the opening of the body lumen may result in a force acting on the opening of the body lumen that is sufficient to cause the opening of the body lumen to adjust to an opened state. The second predetermined flow rate or pressure of the fluid provided to the opening of the body lumen may result in a force acting on the opening and the body lumen that is sufficient to maintain the opening of the lumen in the opened state during a procedure. A lower pressure or flow rate of fluid may be used to maintain the opening of the lumen in the open state than is used to open the opening because the fluid is acting on a greater surface area when maintaining the opening and body lumen in the opened state than a surface area on which the fluid is acting when the fluid is being used to adjust the opening of the body lumen to the opened state. Such use of a lower flow rate, exit velocity or stagnation pressure to maintain the opening of the body lumen in an opened state may result in less fluid being injected into the subject during a procedure than if the same flow rate needed to open the opening of the body lumen were used during the entire procedure, which mitigates injury to the subject.

While the steps illustrated above may provide a method for accessing a body lumen, variations of these methods are also contemplated for achieving the same or a similar goal.

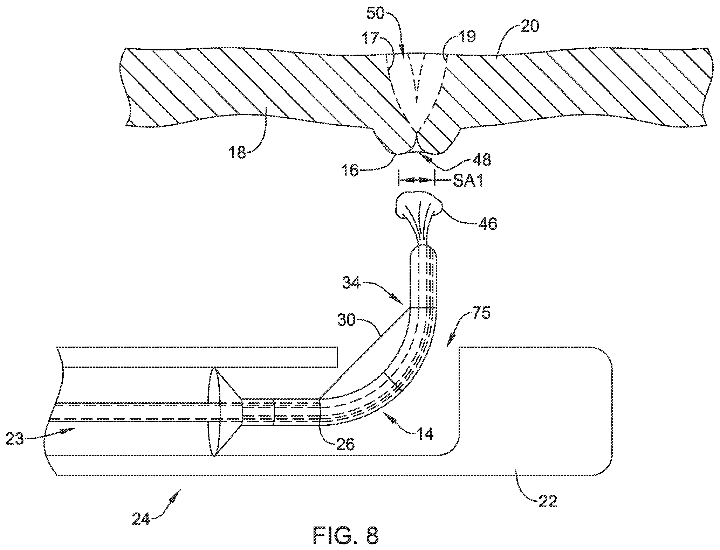

FIGS. 8-10 depict fluid 46 acting on the opening 48 of the major Papilla 16 at the sphincter of Oddi complex (e.g., defining a lumen which leads to the common bile duct 17 and pancreatic duct 19) of a subject to adjust the opening 48 to an opened state from a closed state and maintain the opening 48 in the opened state. The fluid 46 may be provided to the subject from a system such as the system 24 discussed herein and/or from other suitable systems configured to reduce a fluid flow rate from an initial fluid flow rate to a second fluid flow rate while or after adjusting an opening of a body lumen to an opened state and maintain the opening in the opened state using the reduced, second fluid flow rate. Assuming an approximately same or similar force is needed to maintain the opening 48 in the opened state as is needed to open the opening 48, a lower pressure of fluid 46 may be utilized to maintain the opening 48 in the opened state than is needed to open the opening 48, as force equals pressure multiplied by surface area in contact with the pressure.

As depicted in FIG. 8, the medical device 22 (e.g., an endoscope, duodenoscope, etc.) has been positioned in a subject's duodenum with an opening 75 thereof facing the opening 48 of the major Papilla 16, where the opening 48 is in a closed state. The distal tip of the elongate tube 26 of the system 24 may be directed out of the opening 75 of the medical device 22 and fluid 46 may be outputted, at a first predetermined rate, toward the opening 48. The fluid 46 may contact a surface area of the opening 48 in a closed state and must have a pressure sufficient to overcome a natural force of the sphincter of Oddi complex 20 to adjust the opening 48 from the closed state to an opened state. In some cases, the initial flow rate or exit velocity of the fluid 46 (which results in a stagnation pressure at the tissue surface) may be set based on an expected size of the first surface area, but this is not required, so as to create the desired force at the surface area of the closed opening 48 to open the opening 48, while mitigating a volume of used to open the opening 48.

The first surface area may be dependent upon the geometry of the major Papilla 16 in a closed position, as shown in FIG. 8, and the configuration of the fluid 46 exiting the accessory device 14 and contacting the major Papilla 16. To schematically illustrate the first surface area in an example, however, it may be assumed the first surface area can be approximated by a circle having a diameter SA1, as shown in Figure 8. When the circle has a diameter of SA1, the circle may define a surface area of one-half a distance of SA1 (D_{SA1}) squared and multiplied by pi, ~3.14 x (1/2 x D_{SA1})^2.

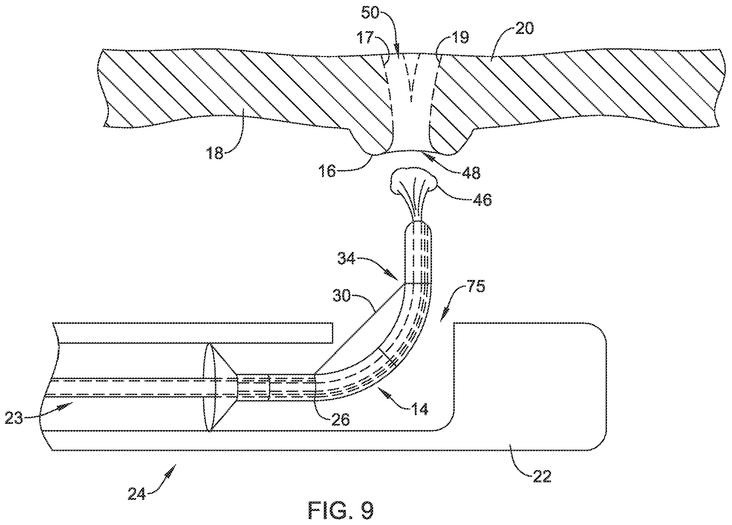

FIG. 9 depicts the opening 48 in an opened state. At this point, the fluid 46 may enter the body lumen 50 and the flow rate of the fluid 46 may have started to decay from the first predetermined flow rate to a second predetermined flow rate, which is slower than the first flow rate and sufficient to maintain the opening 48 of the body lumen 50 in the opened state.

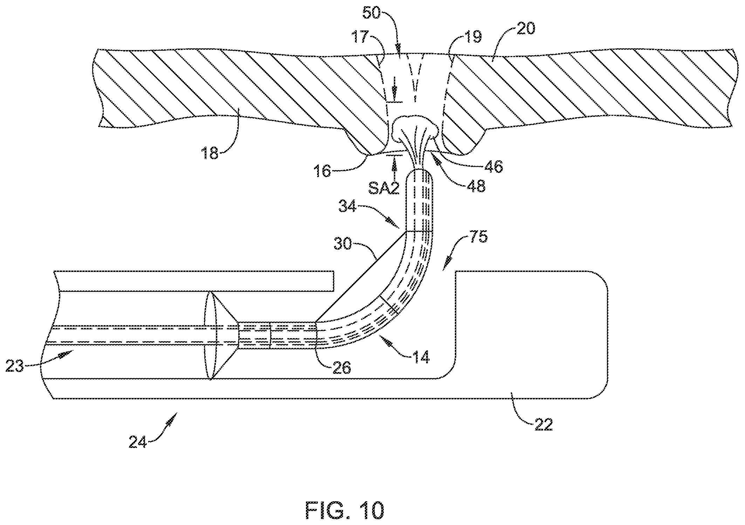

FIG. 10 depicts the opening 48 being maintained in the opened state. As is shown in FIG. 10, the fluid 46 may act on a surface area of the opening 48 and the body lumen 50 along a length SA2 (e.g., a second surface area) to maintain the opening 48 in the opened state during a procedure on the subject. As can be seen when comparing the first surface area on which the fluid 46 acts, as shown in FIG. 8. and the second surface area on which the fluid 46 acts, as shown in FIG. 10, the second surface area (e.g., when the opening 48 is in an opened state) is greater than the first surface (e.g., when the opening 48 is in a closed state. As such, a lower flow rate or fluid pressure at the opening 48 may be utilized to maintain the opening 48 in the opened state than is needed to adjust the opening 48 to the opened state.

The second surface area may be dependent on the geometry of the opening 48 and the body lumen 50 along the length SA2, as shown in FIG. 10, and the configuration of the fluid 46 exiting the accessory device 14 and contacting the major Papilla 16. To schematically illustrate the second surface area in an example, however, it may be assumed the second surface area may be approximated by a bottom-less and top-less cylinder having a length or height of a distance of SA2, D_{SA2}, such that the second surface area may be approximately (D_{SA2}) x a circumference of the opening 48.

### Example of Fluid Flow During a Procedure

In one example configuration of the system 24, carbon dioxide gas (CO₂) may be delivered to a distal end of a sphincterotome catheter in order to access and dilate the major duodenal papilla (Papilla of Vater). In this example, the catheter's CO₂ lumen may have an exit diameter of 0.016 inches and the gas delivery system may be designed to deliver fluid at a first predetermined flow rate and exit velocity out of the catheter when gas flow is initially triggered, followed by a linear decay down to a second predetermined flow rate and exit velocity.

When the flow rate for CO₂ delivery is set to 1.0 liters per minute (lpm) (e.g., 16.67 milliliters/second) at the fluid source, the resulting exiting velocity of the gas leaving the sphincterotome and entering the subject will be 128.5 meters/second (m/s), which is sufficient to adjust the major duodenal papilla to an opened state. When the flow rate for CO₂ delivery is set to 0.5 lpm, the exiting velocity of the gas leaving the sphincterotome and entering the subject will be 64.25 m/s, which is sufficient to maintain the opening at the major duodenal papilla in an opened state.

In some procedures, gas is provided to the major duodenal papilla for forty-five (45) seconds for the purpose of adjusting the papilla to an opened state and maintaining the papilla in the opened state during the procedures. If gas were to be provided to the papilla at the rate that is needed to initially open the papilla (e.g., 1.0 liters per minute in this example) for the full forty-five seconds of gas delivery, 750.15 milliliters of fluid would be delivered to the subject. If gas were to be provided to a subject via a system configured to ramp down a pressure or flow rate of fluid provided to a subject in the manner described herein or otherwise, where a first predetermine flow rate is 1.0 lpm, a second predetermined flow rate is 0.5 lpm, and a decay rate of 0.52 ml/s from the first predetermined flow rate to the second predetermined flow rate, a volume of fluid delivered to the subject over the forty-five seconds of providing fluid to the subject is 441.73 milliliters.

The amount of fluid provided to a subject during forty-five seconds of providing fluid using the tailored decaying flow rate system utilizing the parameters of this example is approximately 58.89% of the volume provided in a system that does not reduce a flow rate after opening the papilla. Additionally, in the ramped down system using a decay rate of 0.52 ml/s, the flow rate is ramped down from the first predetermined flow rate (1.0 lpm) for sixteen seconds and is maintained at the second, non-zero predetermined steady state flow rate (0.5 lpm) for twenty-nine seconds (e.g., which is longer than an amount of time fluid is provided at the first predetermined flow rate), which results in less force acting on (e.g., less trauma on) body tissue coming into contact with the flow of fluid when compared to a constant flow rate system that supplies fluid at a constant rate that is set at a rate needed to create force sufficient to open the opening at the papilla.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A system (24), comprising:
a fluid accumulator (64); and
a sphinctertome (14) in fluid communication with the fluid accumulator (64); and
wherein the fluid accumulator (64) is upstream of the sphinctertome (14) and configured to accumulate pressurized fluid for selective release through a distal end of the sphinctertome at an initial first rate that decays over time to a second rate.

2. The system of claim 1, further comprising:
a pressurized fluid source (40) upstream of and in communication with the fluid accumulator (64) to supply pressurized fluid to the fluid accumulator.

3. The system of claim 2, wherein the second rate is based on a fluid flow rate of the fluid exiting the pressurized fluid source (40).

4. The system of any of claims 2 to 3, further comprising a flow restrictor (62) positioned between the pressurized fluid source (40) and the fluid accumulator (64) to restrict flow from the pressurized fluid source (40) to the fluid accumulator (64).

5. The system of claim 4, further comprising
a fluid source lumen (58) extending from the fluid accumulator (64) and configured to be in fluid communication with the pressurized fluid source (40), wherein the flow restrictor (62) is positioned along the fluid source lumen.

6. The system of any of the preceding claims, wherein the fluid accumulator (64) is a coil of tubing.

7. The system of any of the preceding claims, further comprising:
a valve (68) upstream of the sphinctertome (14) and downstream of the fluid accumulator (64), the valve is configured to adjust a flow of fluid from the fluid accumulator to the sphinctertome.

8. The system of claim 7, further comprising a switch (70) in communication with the valve (68), wherein the switch is configured to be actuated to switch the valve between a closed position in which a fluid flow between the fluid accumulator (64) and the sphinctertome (14) is blocked and an open position in which fluid flows between the fluid accumulator and the sphinctertome.

9. The system of any of claims 7 to 8, wherein the sphinctertome (14) comprises an elongate tube (26) and a connector (74), wherein a lumen of the elongate tube extends from a distal end of the elongate tube toward a proximal end of the elongate tube and through the connector (74) to a flow regulator outlet tubing (72) that is in selective communication with the fluid accumulator (64) via the flow valve (68).

10. The system of claim 9, further comprising
a first tubing portion (66) extending between the fluid accumulator (64) and the flow valve (68).

## Patentansprüche

1. System (24), das aufweist:
einen Fluidspeicher (64); und
ein Sphinkterotom (14), das in Fluidverbindung mit dem Fluidspeicher (64) steht; und
wobei sich der Fluidspeicher (64) stromaufwärts des Sphinkterotoms (14) befindet und
konfiguriert ist, ein unter Druck stehendes Fluid zur selektiven Freisetzung durch ein distales Ende des Sphinkterotoms mit einer anfänglichen ersten Rate zu speichern, die im Laufe der Zeit auf eine zweite Rate abfällt.

2. System nach Anspruch 1, das ferner aufweist:
eine Druckfluidquelle (40), die sich stromaufwärts des Fluidspeichers (64) und in Verbindung mit diesem befindet, um dem Fluidspeicher Druckfluid zuzuführen.

3. System nach Anspruch 2, wobei die zweite Rate auf einer Fluiddurchflussrate des Fluids basiert, das die Druckfluidquelle (40) verlässt.

4. System nach einem der Ansprüche 2 bis 3, das ferner einen Durchflussbegrenzer (62) aufweist, der zwischen der Druckfluidquelle (40) und dem Fluidspeicher (64) angeordnet ist, um den Durchfluss von der Druckfluidquelle (40) zum Fluidspeicher (64) zu begrenzen.

5. System nach Anspruch 4, das ferner aufweist
ein Fluidquellenlumen (58), das sich von dem Fluidspeicher (64) erstreckt und so konfiguriert ist, dass es in Fluidverbindung mit der Druckfluidquelle (40) steht, wobei der Durchflussbegrenzer (62) entlang des Fluidquellenlumens positioniert ist.

6. System nach einem der vorhergehenden Ansprüche, wobei der Fluidspeicher (64) eine Rohrschlange ist.

7. System nach einem der vorhergehenden Ansprüche, das ferner aufweist
ein Ventil (68), das sich stromaufwärts des Sphinkterotoms (14) und stromabwärts des Fluidspeichers (64) befindet, wobei das Ventil konfiguriert ist, einen Fluidstrom von dem Fluidspeicher zu dem Sphinkterotom einzustellen.

8. System nach Anspruch 7, das ferner einen Schalter (70) aufweist, der mit dem Ventil (68) in Verbindung steht, wobei der Schalter konfiguriert ist, betätigt zu werden, um das Ventil zwischen einer geschlossenen Position, in der ein Fluidstrom zwischen dem Fluidspeicher (64) und dem Sphinkterotom (14) blockiert ist, und einer offenen Position, in der Fluid zwischen dem Fluidspeicher und dem Sphinkterotom fließt, umzuschalten.

9. System nach einem der Ansprüche 7 bis 8, wobei das Sphinkterotom (14) ein längliches Rohr (26) und einen Verbinder (74) aufweist, wobei sich ein Lumen des länglichen Rohrs von einem distalen Ende des länglichen Rohrs zu einem proximalen Ende des länglichen Rohrs und durch den Verbinder (74) zu einem Durchflussregler-Auslassrohr (72) erstreckt, das über das Durchflussventil (68) in selektiver Verbindung mit dem Fluidspeicher (64) steht.

10. System nach Anspruch 9, das ferner aufweist
einen ersten Rohrleitungsabschnitt (66), der sich zwischen dem Fluidspeicher (64) und dem Durchflussventil (68) erstreckt.

## Revendications

1. Système (24) comprenant:
un accumulateur de fluide (64); et
un sphinctérotome (14) en communication fluidique avec l'accumulateur de fluide (64);
et
dans lequel l'accumulateur de fluide (64) est en amont du sphinctérotome (14) et configuré pour accumuler un fluide sous pression pour la libération sélective par une extrémité distale du sphinctérotome à un premier débit initial qui diminue au cours du temps jusqu'à un second débit.

2. Système selon la revendication 1, comprenant en outre:
une source de fluide sous pression (40) en amont de et en communication avec l'accumulateur de fluide (64) pour fournir du fluide sous pression à l'accumulateur de fluide.

3. Système selon la revendication 2, dans lequel le second débit est basé sur un débit de fluide du fluide sortant de la source de fluide sous pression (40).

4. Système selon l'une quelconque des revendications 2 à 3, comprenant en outre un limiteur d'écoulement (62) positionné entre la source de fluide sous pression (40) et l'accumulateur de fluide (64) pour limiter l'écoulement de la source de fluide sous pression (40) à l'accumulateur de fluide (64).

5. Système selon la revendication 4, comprenant en outre
une lumière de source de fluide (58) s'étendant depuis l'accumulateur de fluide (64) et configurée pour être en communication fluidique avec la source de fluide sous pression (40), dans lequel le limiteur d'écoulement (62) est positionné le long de la lumière de source de fluide.

6. Système selon l'une quelconque des revendications précédentes, dans lequel l'accumulateur de fluide (64) est un enroulement de tube.

7. Système selon l'une quelconque des revendications précédentes, comprenant en outre:
une vanne (68) en amont du sphinctérotome (14) et en aval de l'accumulateur de fluide (64), la vanne étant configurée pour ajuster un écoulement de fluide de l'accumulateur de fluide au sphinctérotome.

8. Système selon la revendication 7, comprenant en outre un commutateur (70) en communication avec la vanne (68), dans lequel le commutateur est configuré pour être actionné pour commuter la vanne entre une position fermée dans laquelle un écoulement de fluide entre l'accumulateur de fluide (64) et le sphinctérotome (14) est bloqué et une position ouverte dans laquelle du fluide s'écoule entre l'accumulateur de fluide et le sphinctérotome.

9. Système selon l'une quelconque des revendications 7 à 8, dans lequel le sphinctérotome (14) comprend un tube allongé (26) et un connecteur (74), dans lequel une lumière du tube allongé s'étend d'une extrémité distale du tube allongé vers une extrémité proximale du tube allongé et à travers le connecteur (74) jusqu'à un tube de sortie de régulateur d'écoulement (72) qui est en communication sélective avec l'accumulateur de fluide (64) par l'intermédiaire de la vanne d'écoulement (68).

10. Système selon la revendication 9, comprenant en outre une première portion de tube (66) s'étendant entre l'accumulateur de fluide (64) et la vanne d'écoulement (68).
